# EUROPEAN PATENT SPECIFICATION

(11) **EP 1 149 912 B1**
(45) Date of publication and mention of the grant of the patent: **26.05.2010**
(21) Application number: 01108169.2
(22) Date of filing: 30.03.2001
(51) Int. Cl.: C12N 15/54, C12N 5/10, C12N 9/10, C12P 7/62

(54) **Polyhydroxyalkanoate synthase and gene encoding the same**
Polyhydroxyalkanoat-Synthase und dieses Enzym kodierendes Gen
Polyhydroxyalcanoate synthase et gène codant pour cette enzyme

(30) Priority: 30.03.2000 JP 2000095005
(43) Date of publication of application: 31.10.2001
(73) Proprietor: CANON KABUSHIKI KAISHA, Tokyo (JP)
(72) Inventor: Yano, Tetsuya, Ohta-ku, Tokyo (JP); Imamura, Takeshi, Ohta-ku, Tokyo (JP); Suda, Sakae, Ohta-ku, Tokyo (JP); Honma, Tsutomu, Ohta-ku, Tokyo (JP)
(74) Representative: Weser, Wolfgang

(56) References cited:
- EP-A- 0 897 005
- WO-A-01/11014
- WO-A-91/00917
- US-A- 5 849 894
- MATSUSAKI HIROMI ET AL: "Cloning and molecular analysis of the poly(3-hydroxybutyrate) and poly(3-hydroxybutyrate-co-3-hydroxyalkanoa te) biosynthesis genes in Pseudomonas sp. strain 61-3" JOURNAL OF BACTERIOLOGY, WASHINGTON, DC, US, vol. 180, no. 24, December 1998 (1998-12), pages 6459-6467, XP002167124 ISSN: 0021-9193 -& DATABASE SWALL [Online] SWISSPROT; AC: Q9Z3X9, 1 May 1999 (1999-05-01) MATSUSAKI ET AL.: "PHA synthase 2" XP002176384 -& DATABASE SWALL [Online] SWISSPROT; AC:Q9Z3Y1, 1 May 1999 (1999-05-01) MATSUSAKI ET AL.: "PHA synthase 1" XP002176385 -& DATABASE EM_PRO [Online] EMBL; AC:AB014758, 29 December 1998 (1998-12-29) MATSUSAKI ET AL.: "Pseudomonas sp. 61-3 genes" XP002176386
- HUISMAN G W ET AL: "METABOLISM OF POLY-3-HYDROXYALKANOATES PHAS BY PSEUDOMONAS-OLEOVORANS IDENTIFICATION AND SEQUENCES OF GENES AND FUNCTION OF THE ENCODED PROTEINS IN THE SYNTHESIS AND DEGRADATION OF PHA" JOURNAL OF BIOLOGICAL CHEMISTRY, vol. 266, no. 4, 1991, pages 2191-2198, XP002176382 ISSN: 0021-9258
- GARCIA BELEN ET AL: "Novel biodegradable aromatic plastics from a bacterial source: Genetic and biochemical studies on a route of the phenylacetyl-CoA catabolon." JOURNAL OF BIOLOGICAL CHEMISTRY, vol. 274, no. 41, 8 October 1999 (1999-10-08), pages 29228-29241, XP002176383 ISSN: 0021-9258

### SEQUENCE LISTING

<110>Canon INC.
<120>Polyhydroxyalkanoate synthase and gene encoding the same
<121>
<130>4051022
<160>10
<210>1
<211>559
<212>PRT
<213>Pseudomonas putida P91
<220>
<223>Polyhydroxyalkanoate synthase
<400>1
   Met Ser Asn Lys Asn Asn Asp Asp Leu Gln Arg Gln Ala Ser Glu Asn
   1 5 10 15
   Thr Leu Gly Leu Ser Pro Ile Ile Gly Leu Arg Arg Lys Asp Leu Leu
   20 25 30
   Ser Ser Ala Arg Met Val Leu Arg Gln Ala Ile Lys Gln Pro Leu His
   35 40 45
   Ser Ala Lys His Val Ala His Phe Gly Leu Gln Leu Lys Asp Val Ile
   50 55 60
   Phe Gly Lys Ser Gly Leu Gln Pro Glu Gly Asp Asp Arg Arg Phe Ser
   65 70 75 80
   Asp Pro Ala Trp Ser Gln Asn Pro Leu Tyr Arg Arg Tyr Leu Gln Thr
   85 90 95
   Tyr Leu Ala Trp Arg Lys Glu Leu His Asp Trp Ile Gly Asn Ser Asn
   100 105 110
   Leu Ser Glu Gln Asp Ile Ser Arg Ala His Phe Val Ile Asn Leu Met
   115 120 125
   Thr Glu Ala Met Ala Pro Thr Asn Ser Ala Ala Asn Pro Ala Ala Val
   130 135 140
   Lys Arg Phe Phe Glu Thr Gly Gly Lys Ser Leu Leu Asp Gly Leu Ser
   145 150 155 160
   His Leu Ala Lys Asp Met Val His Asn Gly Gly Met Pro Ser Gln Val
   165 170 175
   Asn Met Asp Ala Phe Glu Val Gly Lys Asn Leu Ala Thr Thr Glu Gly
   180 185 190
   Ala Val Val Phe Arg Asn Asp Val Leu Glu Leu Ile Gln Tyr Arg Pro
   195 200 205
   Ile Thr Glu Gln Val His Glu Lys Pro Leu Leu Val Val Pro Pro Gln
   210 215 220
   Ile Asn Lys Phe Tyr Val Phe Asp Leu Ser Pro Glu Lys Ser Leu Ala
   225 230 235 240
   Arg Phe Cys Leu Arg Ser Thr Val Gln Thr Phe Ile Val Ser Trp Arg
   245 250 255
   Asn Pro Asn Lys Ser Gln Arg Glu Trp Gly Leu Ser Thr Tyr Ile Asp
   260 265 270
   Ala Leu Lys Glu Ala Val Asp Val Val Leu Ala Ile Thr Gly Ser Lys
   275 280 285
   Asp Leu Asn Met Leu Gly Ala Cys Ser Gly Gly Ile Thr Cys Thr Ala
   290 295 300
   Leu Val Gly His Tyr Ala Ala Leu Gly Glu Lys Lys Val Asn Ala Leu
   305 310 315 320
   Thr Leu Leu Val Ser Val Leu Asp Thr Thr Leu Asp Thr Gln Val Ala
   325 330 335
   Leu Phe Val Asp Glu Gln Thr Leu Glu Ser Ala Lys Arg His Ser Tyr
   340 345 350
   Gln Ala Gly Val Leu Glu Gly Arg Asp Met Ala Lys Val Phe Ala Trp
   355 360 365
   Met Arg Pro Asn Asp Leu Ile Trp Asn Tyr Trp Val Asn Asn Tyr Leu
   370 375 380
   Leu Gly Asn Glu Pro Pro Val Phe Asp Ile Leu Phe Trp Asn Asn Asp
   385 390 395 400
   Ile Thr Arg Leu Pro Ala Ala Phe His Gly Asp Leu Ile Glu Met Phe
   405 410 415
   Lys Asn Asn Pro Leu Val Arg Pro Gly Ala Leu Glu Val Cys Gly Thr
   420 425 430
   Pro Ile Asp Leu Ser Gln Val Thr Thr Asp Ile Phe Ser Val Ala Gly
   435 440 445
   Thr Asn Asp His Ile Thr Pro Trp Lys Ser Cys Tyr Lys Ser Ala Gln
   450 455 460
   Leu Phe Gly Gly Lys Val Glu Phe Leu Leu Ser Asn Ser Gly His Ile
   465 470 475 480
   Gln Ser Ile Leu Asn Pro Pro Gly Asn Pro Lys Ser Arg Tyr Met Thr
   485 490 495
   Ser Ser Glu Met Pro Ala Gln Ala Asp Asp Trp Gln Glu Asn Ser Thr
   500 505 510
   Lys His Thr Asp Ser Trp Trp Leu Tyr Trp Gln Ala Trp Leu Ala Glu
   515 520 525
   Arg Ser Gly Ala Leu Lys Pro Ala Pro Ala Lys Leu Gly Asn Lys Ala
   530 535 540
   Tyr Pro Ser Ala Glu Ala Ser Pro Gly Thr Tyr Val His Glu Arg
   545 550 555
<210>2
<211>1680
<212>DNA
<213>Pseudomonas putida P91
<220>
<221>CDS
<222>(1)...(1680) Polyhydroxyalkanoate synthase encoding sequence
<400>2
   atgagtaaca agaacaacga tgacctgcag cgccaagcct ctgaaaacac cctgggcctg 60
   agccccatca ttggcctgcg ccgaaaggat ttgctgtctt cggcccggat ggtgctgcgt 120
   caggccatca agcaaccgct gcacagtgcc aagcacgtcg cgcatttcgg cctgcagctc 180
   aaggacgtga tcttcggcaa gtccggcctg cagccggagg gcgacgaccg ccgcttcagc 240
   gacccggcct ggagccagaa cccgctgtac cgccgctacc tgcagaccta cctggcctgg 300
   cgcaaggaac tgcacgactg gatcggcaac agcaacctgt cggagcagga catcagccgc 360
   gcgcacttcg tcatcaacct gatgaccgag gccatggccc ccaccaacag cgcggccaac 420
   ccggcagcgg tcaagcgctt cttcgaaacc ggtggcaaga gcctgctcga cggcctgtcg 480
   cacctggcca aggacatggt ccacaacggc ggcatgccca gccaggtcaa catggacgcc 540
   ttcgaggtgg gcaagaacct ggccaccacc gagggcgccg tggtatttcg caacgacgtg 600
   ctggagctga tccagtaccg cccgatcacc gagcaggtgc acgaaaagcc gctgctggtg 660
   gtaccgccgc agatcaacaa gttctacgtc ttcgacctca gcccggaaaa gagcctggcg 720
   cgcttctgcc tgcgctccac ggtgcagacc ttcatcgtga gctggcgcaa ccccaacaag 780
   tcccagcgcg agtggggcct gtcgacctac atcgatgcgc tcaaggaggc cgtcgacgtg 840
   gtgctggcaa tcaccggcag caaggacctg aacatgctcg gtgcctgctc cggcggcatc 900
   acctgcaccg cgctggtggg ccactacgcg gcactgggcg agaagaaggt caatgccctg 960
   accctgctgg tgagcgtgct cgacaccacc ctcgacaccc aggtggcgct gttcgtcgac 1020
   gagcagaccc tggagtcggc caagcgccat tcctaccagg ccggtgtgct cgaaggccgc 1080
   gacatggcca aggtgttcgc ctggatgcgc cccaacgacc tgatctggaa ctactgggtc 1140
   aacaactacc tgctcggcaa cgagccgccg gtgttcgaca tcctgttctg gaacaacgac 1200
   atcacgcgcc tgcccgccgc cttccacggc gacctgatcg aaatgttcaa gaacaacccg 1260
   ctggtgcgtc ccggtgcact ggaagtgtgc ggcacgccga tcgacctgag ccaggtcacc 1320
   accgacatct tcagcgtggc cggcaccaac gatcacatca ccccatggaa gtcctgctac 1380
   aagtcggcgc agctgttcgg cggcaaggtc gagttcctgc tgtccaacag cgggcatatc 1440
   cagagcatcc tcaacccgcc gggcaacccc aagtcgcgct acatgaccag cagcgagatg 1500
   ccggcccagg ccgacgactg gcaggagaac tccaccaagc acaccgattc ctggtggctg 1560
   tactggcagg cgtggctggc cgagcgctcc ggcgcactca agccggcacc cgccaagctg 1620
   ggcaacaagg cctacccgag cgccgaagcg tcgcccggca cctacgtcca cgaacgctga 1680
<210>3
<211>560
<212>PRT
<213>Pseudomonas putida P91
<220>
<223>Polyhydroxyalkanoate synthase
<400>3
   Met Lys Asp Lys Pro Ala Lys Pro Gly Val Pro Thr Pro Ala Ala Tyr
   1 5 10 15
   Leu Asn Val Arg Ser Ala Ile Ser Gly Leu Arg Gly Arg Asp Leu Leu
   20 25 30
   Ser Thr Val His Gln Leu Gly Arg His Gly Leu Arg His Pro Leu His
   35 40 45
   Thr Ala Arg His Leu Leu Ala Leu Gly Gly Gln Leu Gly Arg Val Met
   50 55 60
   Leu Gly Asp Thr Pro Tyr Gln Pro Ser Pro Arg Asp Thr Arg Phe Asn
   65 70 75 80
   Asp Pro Ala Trp Gln Leu Asn Pro Leu Tyr Arg Arg Gly Leu Gln Ala
   85 90 95
   Tyr Leu Ala Trp Gln Gln Gln Thr Cys Gln Trp Ile Asp Glu Ser Gln
   100 105 110
   Leu Asp Asp Asp Asp Arg Ala Arg Ala His Phe Val Phe Ser Leu Leu
   115 120 125
   Asn Asp Ala Met Ser Pro Ser Asn Thr Leu Leu Asn Pro Ala Ala Val
   130 135 140
   Lys Glu Leu Leu Asn Ser Gly Gly Leu Ser Leu Val Arg Gly Leu Asn
   145 150 155 160
   His Leu Leu Asp Asp Leu Arg His Asn Asp Gly Leu Pro Arg Gln Val
   165 170 175
   Asn Pro Asp Ala Phe Glu Val Gly Arg Asn Leu Ala Ser Thr Ala Gly
   180 185 190
   Ala Val Val Phe Arg Asn Glu Leu Leu Glu Leu Ile Gln Tyr Arg Pro
   195 200 205
   Met Ser Glu Lys Gln Tyr Ala Arg Pro Leu Leu Val Val Pro Pro Gln
   210 215 220
   Ile Asn Lys Phe Tyr Ile Phe Asp Leu Ser Pro Thr Asn Ser Phe Val
   225 230 235 240
   Gln Tyr Ala Leu Lys Asn Gly Leu Gln Thr Phe Met Ile Ser Trp Arg
   245 250 255
   Asn Pro Asp Ala Arg His Arg Glu Trp Gly Leu Ser Ser Tyr Val Ala
   260 265 270
   Ala Val Glu Glu Ala Met Asn Val Cys Arg Ser Ile Thr Gly Ser Arg
   275 280 285
   Asp Val Asn Leu Leu Gly Ala Cys Ala Gly Gly Leu Thr Ile Ala Ala
   290 295 300
   Leu Gln Gly His Leu Gln Ala Lys Arg Gln Met Arg Arg Val His Ser
   305 310 315 320
   Ala Thr Tyr Leu Val Ser Leu Leu Asp Ser Gln Phe Asp Ser Pro Ala
   325 330 335
   Ser Leu Phe Ala Asp Glu Gln Thr Leu Glu Ala Ala Lys Arg Arg Ser
   340 345 350
   Tyr Gln Gln Gly Val Leu Glu Gly Arg Glu Met Ala Arg Val Phe Ala
   355 360 365
   Trp Met Arg Pro Asn Asp Leu Ile Trp Asn Tyr Phe Val Asn Asn Tyr
   370 375 380
   Leu Leu Gly Lys Ala Pro Pro Ala Phe Asp Ile Leu Tyr Trp Asn Asn
   385 390 395 400
   Asp Asn Ser Arg Leu Pro Ala Ala Leu His Gly Asp Leu Leu Asp Phe
   405 410 415
   Phe Lys Phe Asn Pro Leu Thr His Ala Asp Gly Leu Glu Val Cys Gly
   420 425 430
   Thr Pro Ile Asp Leu Asn Lys Val Thr Val Asp Ser Phe His Val Ala
   435 440 445
   Gly Ser Asn Asp His Ile Thr Pro Trp Asp Ala Val Tyr Arg Ser Ala
   450 455 460
   Leu Leu Leu Gly Gly Glu Arg Arg Phe Val Leu Ala Asn Ser Gly His
   465 470 475 480
   Val Gln Ser Ile Leu Asn Pro Pro Gly His Pro Lys Ala His Phe Val
   485 490 495
   Glu Asn Pro Arg Leu Ser Ser Asp Pro Arg Ala Trp Tyr His Asp Ala
   500 505 510
   Gln Lys Val Glu Gly Ser Trp Trp Pro Gln Trp Leu Asp Trp Ile Gln
   515 520 525
   Ala Arg Ser Gly Ala Gln Arg Glu Thr Arg Leu Ser Leu Gly Ser Ala
   530 535 540
   Asn Tyr Pro Pro Met Asp Pro Ala Pro Gly Thr Tyr Val Leu Val Arg
   545 550 555 560
<210>4
<211>1683
<212>DNA
<213>Pseudomonas putida P91
<220>
<221>CDS
<222>(1)...(1683) Polyhydroxyalkanoate synthase encoding sequence
<400>4
   atgaaagaca agcccgcgaa gcccggggta ccgacccccg ctgcctatct caacgtgcgc 60
   agcgccatca gtggcctgcg cggtcgcgac ctgctgtcga cggtgcacca gctggggcgc 120
   cacggcctgc gtcacccgct gcacacggcg cgccacctgc tggcgctggg tggccagctg 180
   gggcgcgtga tgctgggcga taccccctac cagccctcgc cacgcgacac ccgcttcaac 240
   gacccggcct ggcagctcaa cccgctgtac cgacgcggcc tgcaggccta cctggcctgg 300
   cagcagcaga cctgccagtg gatcgacgag agccagctgg acgacgatga ccgcgcccgc 360
   gcgcacttcg tgttctcgct gctcaacgat gcaatgtcgc ccagcaacac cctgctcaac 420
   ccggcggcgg tcaaggagct gctgaactcc ggcgggctga gcctggtgcg cggcttgaac 480
   cacctgctcg acgacctgcg ccacaacgac ggcctgccac gccaggtcaa cccggacgcc 540
   ttcgaggtgg gcaggaacct ggccagcacc gccggcgcgg tggtgtttcg caacgagctg 600
   ctggagctga tccagtaccg cccgatgagc gaaaaacagt acgcccggcc cctgctggtg 660
   gtgccgccgc agatcaacaa gttctacatc ttcgacctca gcccgaccaa cagctttgtg 720
   cagtacgccc tcaagaacgg cctgcagacc ttcatgatca gctggcgcaa ccccgacgcc 780
   cggcatcgcg aatggggcct gtcgagctac gtggcggcgg tcgaggaagc catgaacgtg 840
   tgccgctcga tcaccggcag ccgcgacgtc aacctgcttg gcgcctgtgc cggcgggttg 900
   accatcgcgg ccctgcaggg tcacctgcag gccaagcgcc agatgcgccg ggtgcacagc 960
   gccacctacc tggtcagcct gctcgacagc cagttcgaca gccccgccag cctgttcgcc 1020
   gacgagcaga ccctggaggc ggccaagcgc cgctcctacc agcagggcgt gctggagggc 1080
   cgcgagatgg cacgggtgtt cgcctggatg cgccccaacg acctgatctg gaactacttc 1140
   gtcaacaact acctgctggg caaggcgccc ccggcattcg acatcctgta ctggaacaac 1200
   gacaacagcc gcctgccggc cgcgctgcac ggcgatctgc tggacttctt caaattcaac 1260
   ccgctgacgc acgccgacgg cctcgaggta tgcggcacgc cgatcgacct gaacaaggtc 1320
   acggtggaca gcttccacgt ggccggcagc aacgaccaca tcaccccgtg ggacgcggtg 1380
   taccgctcgg ccctgctgct gggcggcgag cggcgcttcg tgctggccaa cagcgggcat 1440
   gtgcagagca tcctcaaccc accgggccac cccaaggcgc attttgtcga gaaccccagg 1500
   ctgagcagcg acccgcgggc ctggtaccac gatgcgcaga aggtcgaggg cagctggtgg 1560
   ccgcagtggc tcgactggat acaggcgcgc tccggtgcgc agcgcgaaac ccgcctgtcg 1620
   ctgggcagcg ccaattaccc tcccatggac cccgcacccg gcacctacgt gctggtgcgc 1680
   tga 1683
<210>5
<211>20
<212>DNA
<220>
<223>Probe sequence
<400>5
   tgctggaact gatccagtac 20.
<210>6
<211>23
<212>DNA
<220>
<223>Probe sequence
<400>6
   gggttgagga tgctctggat gtg 23
<210>7
<211>30
<212>DNA
<220>
<223>Primer sequence for PCR
<400>7
   cagccaagct tgtactcgtc tcaggacaac 30
<210>8
<211>29
<212>DNA
<220>
<223>Primer sequence for PCR
<400>8
   agagataagc ttgcggcatg cgcgagccc 29
<210>9
<211>30
<212>DNA
<220>
<223>Primer sequence for PCR
<400>9
   cattgaagct ttggttgatg gcctgacgac 30
<210>10
<211>29
<212>DNA
<220>
<223>Primer sequence for PCR
<400>10
   ctccaagctt cggtcgcggg tcttcatcc 29

## Description

### BACKGROUND OF THE INVENTION

### Field of the Invention

This invention relates to a polyhydroxyalkanoate (hereinafter, referred to as a "PHA") synthase, a gene encoding the synthase, a recombinant vector containing the gene, a transformant transformed by the vector, a process for producing the PHA synthase utilizing the transformant, and a process for preparing the PHA utilizing the transformant.

### Related Background Art

There have been reported a number of microorganisms producing poly-3-hydroxybutyric acid (PHB) or another PHA and storing it therein ("Biodegradable Plastic Handbook", edited by Biodegradable Plastic Research Society, NTS Co. Ltd., p. 178-197 1995). These polymers may be, as conventional plastics, used for producing a variety of products by, for example, melt-processing. Since they are biodegradable, they have an advantage that they can be completely degraded by microorganisms in the natural environment, and they do not cause pollution due to remaining in the natural environment like many conventional polymer compounds. Furthermore, they are excellently biocompatible, and thus are expected to be used in applications such as a medical soft member.

It is known that a composition and a structure of such a PHA produced by a microorganism may considerably vary depending on the type of a microorganism used for the production, a culture-medium composition and culturing conditions. Investigations have been, therefore, mainly focused on controlling such a composition or structure for the purpose of improving physical properties of a PHA.

For example, Japanese Patent Application Laid-Open No. 6-15604, No. 7-14352 and No. 8-19227 have described that Alcaligenes eutropus H16 (ATCC No. 17699) and its variants may produce 3-hydroxybutyric acid (3HB) and its copolymer with 3-hydroxyvaleric acid (3HV) with various composition ratios by changing a carbon source during culturing.

Japanese Patent Publication No. 2642937 has disclosed that PHA in which a monomer unit is 3-hydroxyalkanoate with 6 to 12 carbon atoms may be produced by supplying a non-cyclic aliphatic hydrocarbon as a carbon source to Pseudomonas oleovorans (ATCC No. 29347).

Japanese Patent Application Laid-Open No. 5-74492 has disclosed methods in which Methylobaterium sp., Paracoccus sp., Alcaligenes sp. and Pseudomonas sp. are contacted with a primary alcohol with 3 to 7 carbon atoms to produce a copolymer of 3HB and 3HV.

Japanese Patent Application Laid-Open No. 5-93049 and No. 7-265065 have disclosed that Aeromonas caviae is cultured using oleic acid or olive oil as a carbon source to produce a two-component copolymer of 3HB and 3-hydroxyhexanoic acid (3HHx).

Japanese Patent Application Laid-Open No. 9-191893 has disclosed that Comamonas acidovorans IF013852 is cultured using gluconic acid and 1,4-butanediol as carbon sources to produce a polyester having 3HB and 4-hydroxybutyric acid as monomer units.

Furthermore, it has been reported that certain microorganisms produce PHAs having a variety of substituents such as groups derived from an unsaturated hydrocarbon, ester, allyl, cyano, groups derived from a halogenated hydrocarbon and epoxide. Recently, there have been attempts for improving physical properties of a PHA produced by a microorganism using such a procedure.

As an example of such a polymer, a PHA having a phenyl group in its side chain has been developed. For example, Makromol. Chem., 191, 1957-1965 (1990); Macromolecules, 24, 5256-5260 (1991); and Chirality, 3, 492-494 (1991) have described production of a PHA comprising 3-hydroxy-5-phenylvaleric acid (3HPV) as a monomer unit by Pseudomonas oleovorans, where there has been observed variation in polymer physical properties probably due to the presence of 3HPV.

EP 0 897 005 discloses a polypeptide having polyester synthase activity and comprising a specified amino acid sequence or variants thereof obtained by deletion, replacement or addition of one or more amino acids. Further disclosed are a polyester synthase gene comprising DNA coding for the polypeptide, and a recombinant vector comprising the gene. B. Garcia et al. describe synthesis of polymers containing units of 3-hydroxy-n-phenylalkanoic acids by Pseudomonas putida U (in J. Biol. Chem. 274 (1999), pp29228-29241).

And G. Huisman et al. report on metabolism of poly(3-hydroxyalkanoates) (PHAs) by Pseudomonas oleovorans (in J. Biol. Chem. 266 (1991), pp2191-2198).

As described above, microorganism-produced PHAs with various combinations of composition and structure have been obtained by varying factors such as the type of a microorganism used, a culture medium composition and culturing conditions. However, each microorganism or PHA synthase has significantly different substrate specificity. Therefore, it has been difficult to produce PHAs comprising different monomer units extensively suitable to a variety of applications using known microorganisms or PHA synthases alone.

### SUMMARY OF THE INVENTION

A PHA having a substituent in its side chain as described above may be expected to be a "functional polymer" having significantly useful functions and properties owing to the properties of the introduced substituent. It is, therefore, extremely useful and important to prepare a gene encoding a PHA synthase from a microorganism which can produce and store a very useful polymer having both such functionality and biodegradability; prepare a recombinant vector comprising the gene, a transformant transformed by the vector; and develop a process for producing a PHA synthase utilizing the transformant and a process for preparing a PHA utilizing the transformant.

In view of usefulness of such a PHA synthase useful in PHA production, an object of the present invention is to provide a PHA synthase, a gene encoding the enzyme, a recombinant vector comprising the gene, a transformant transformed by the vector, a process for producing a PHA synthase utilizing the transformant and a process for preparing a PHA utilizing the transformant.

The above objects are achieved by the polyhydroxyalkanoate synthase according to claims 1, 2, 3, 6, 7, and 8, the polyhydroxyalkanoate synthase gene according to claims 4, 5, 9, and 10, the recombinant vector according to claim 11, the transformed microorganism according to claim 12, the method for preparing polyhydroxyalkanoate according to claim 13, and the method for producing a polyhydroxyalkanoate synthase according to claim 14.

For developing a PHA having a novel side-chain structure useful as, for example, a device material or a medical material, the inventors have searched a novel microorganism capable of producing and storing the desired PHA. Additionally, the inventors have intensely investigated for preparing a gene encoding a PHA synthase from such a microorganism, a recombinant vector containing the gene, a transformant transformed by the vector and developing a process for producing a PHA synthase utilizing the transformant and a process for preparing a PHA utilizing the transformant.

The inventors have finally found a novel microorganism capable of producing and storing a novel PHA comprising 3-hydroxy-5-(4-fluorophenyl)valeric acid (3HFPV) represented by formula (2) as a monomer unit from synthetic 5-(4-fluorophenyl)valeric acid (FPVA) represented by formula (1) as a starting material, and designate it as P91 strain.

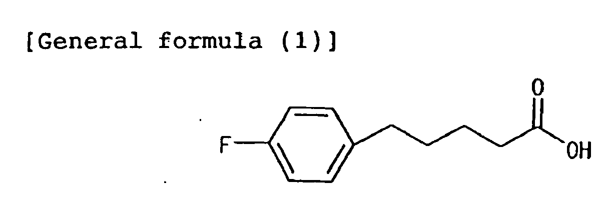

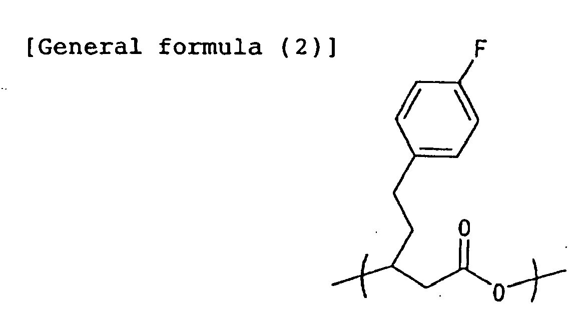

The inventors have also found that P91 strain in capable of producing and storing a PHA comprising 3-hydroxy-4-phenoxy-n-butyric acid (3HPxB) represented by formula (4) as a monomer unit from 4-phenoxy-n-butyric acid (PxBA) represented by formula (3) as a starting material.

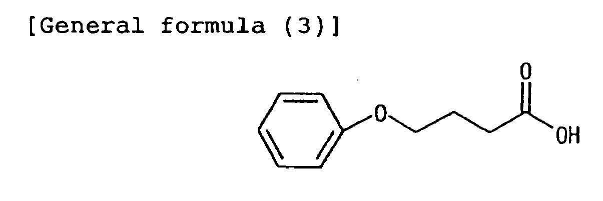

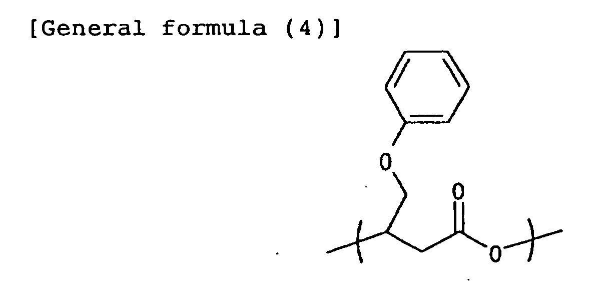

An example of a microorganism capable of producing and storing a PHA comprising 3HPxB as a monomer unit is Pseudomonas oleovorans involved in a process described in Macromolecules, 29, 3432-3435, 1996. This process is considerably different from the process using PxBA as a substrate in P91 strain in that 8-phenoxyoctanoic acid (PxOA) is used as a substrate. In addition, for a PHA produced, the above reported process provides a copolymer consisting of three monomer units, i.e., 3-hydroxy-8-phenoxyoctanoic acid derived from the substrate PxOA, 3-hydroxy-6-phenoxyhexanoic acid as a byproduct derived from a metabolite and 3HPxB. On the other hand, P91 strain can produce a PHA comprising 3HPxB derived from PxBA as a sole phenoxy-containing monomer unit. In this respect, P91 strain is basically different from the above reported strain.

There are no reports describing microbial production of a PHA comprising 3HPxB as a monomer unit using PxBA as a substrate or 3HPxB as a sole phenoxy-containing monomer unit.

Microbiological properties of P91 strain according to this invention are as follows.

### <Microbiological properties of P91 strain>

### (Morphologic properties)

Cell shape and size: Bacilliform, 0.6 µm × 1.5 µm
Cell polymorphism: No
Motility: Yes
Sporulation: No
Gram stainability: Negative
Colonization: Circular, smooth in the overallperiphery, low convex, smooth surface, gloss, cream color

### (Physiological properties)

Catalase: Positive
Oxidase: Positive
O/F test: oxidized form
Reduction of a nitrate: Negative
Indole formation: Negative
Acidification of dextrose: Negative
Arginine dihydrolase: Positive
Urease: Negative
Esculin hydrolysis: Negative
Gelatin hydrolysis: Negative
β-Galactosidase: Negative
Fluorochrome production on King's B agar: Positive

### (Substrate assimilation ability)

Dextrose: Positive
L-Arabinose: Negative
D-Mannose: Negative
D-Mannitol: Negative
N-Acetyl-D-glucosamine: Negative
Maltose: Negative
Potassium gluconate: Positive
n-Capric acid: Positive
Adipic acid: Negative
dl-Malic acid: Positive
Sodium citrate: Positive
Phenyl acetate: Positive

From these microbiological properties, the inventors have attempted to categorize P91 strain according to Bergey's Manual of Systematic Bacteriology, Volume 1 (1984) and Bergey's Manual of Determinative Bacteriology 9th ed. (1994) to determine that the strain belongs to Pseudomonas putida. Thus, the strain was designated as Pseudomonas putida P91. The inventors have deposited Pseudomonas putida P91 to Patent Microorganism Depository Center in the National Institute of Bioscience and Human Technology, Agency of Industrial Science and Technology, Ministry of International Trade and Industry, under the deposition number of FERM P-17409. P91 strain has been internationally deposited on the basis of the Budapest Treaty, and its international deposition number is "FERM BP-7373".

The inventors have intensely conducted investigation for solving the above problems and finally have succeeded cloning a gene for a PHA synthase from P91 strain to achieve this invention.

Specifically, a PHA synthase of this invention is characterized in that it has the amino acid sequence of SEQ ID NO.:1 or 3. A PHA synthase according to the present invention may include a mutant PHA synthase where at least one mutation including deletion, substitution or addition of at least one amino acid is introduced as long as it does not deteriorate PHA synthase activity exhibited by a protein comprising these amino acid sequences.

The present invention also encompasses a PHA synthase gene coding a PHA synthase comprising the amino acid sequence of SEQ ID NO.:1 or 3. Examples of a sequence of such a gene include SEQ ID NO.:2 or 4. Furthermore, a mutant PHA synthase gene encoding the above mutant PHA synthase obtained by mutation of the sequence of SEQ ID NOs.:2 and 4 is included in a PHA synthase gene according to this invention.

The present invention also encompasses a recombinant vector comprising the above PHA synthase gene and a transformant transformed by the recombinant vector. The present invention also encompasses a process for producing a PHA synthase comprising the steps of culturing the transformant and isolating the PHA synthase from a culture obtained, and a process for preparing a PHA comprising the steps of culturing the transformant and isolating the PHA from a culture obtained.

The present invention provides a PHA synthase, a gene encoding the PHA synthase, a recombinant vector comprising the gene and a transformant transformed by the recombinant vector. The PHA synthase gene according to the present invention is useful for preparing a PHA having various physical properties because it encodes a PHA synthase using a monomer having a novel side-chain structure as a substrate.

### DETAILED DESCRIPTION OF THE PREFERRED EMBODIMENTS

The present invention will be more detailed. A PHA synthase gene of the present invention is isolated from Pseudomonas putida P91 strain. First, a chromosome DNA is obtained from a strain having a PHA synthase gene. The chromosome DNA may be isolated by a known technique.

For example, after P91 strain is cultured in a LB medium or an M9 medium supplemented with an appropriate carbon source, a chromosome DNA is prepared as described by, for example, Marmer et al. in Journal of Molecular Biology, Vol. 3, p. 208 (1961). The chromosome DNA thus obtained is digested using an appropriate restriction enzyme (e.g., Sau3AI) and a fragment with a proper length is ligated with a ligatable vector digested with a restriction enzyme (e.g., BamHI) to prepare a gene library. Herein, a proper fragment length varies, e.g., about 4000 to 25000 bps for a usual plasmid vector and about 15000 to 30000 bps for a cosmid or phage vector. A proper length of DNA fragment may be collected by a known method such as a method using a sucrose density gradient or using an agarose gel described in Molecular Cloning, Cold Spring Harbor Laboratory (1982).

A vector is a phage vector or plasmid vector which can autonomously replicate in the host microorganism. Examples of phage or cosmid vectors include pWE15, M13, λEMBL3, λEMBL4, λFIXII, λDASHII, λZAPII, λgt10, λgt11, Charon4A and Charon21A. Examples of plasmid vectors include pBR, pUC, pBluescriptII, pGEM, pTZ and pET groups. Various shuttle vectors may be used, e.g., vectors which may autonomously replicate in a plurality of host microorganisms such as E. coli and Pseudomonas sp. These vectors may be also digested with a proper restriction enzyme to provide a desired fragment as described above.

A chromosome DNA fragment may be ligated with a vector fragment using a DNA ligase. For example, a ligation kit (Takara Shuzo Co., Ltd., etc.) may be used. Thus, for example, a chromosome DNA fragment may be ligated with a vector fragment to prepare a mixture of recombinant plasmids comprising various DNA fragments (hereinafter, referred to as a "gene library"). In addition to a method using a proper length of chromosome DNA fragment, a gene library may be prepared by a method that mRNAs are extracted from P91 strain, purified and used for preparation of a cDNA fragment using a reverse transcriptase as described in Molecular Cloning, Cold Spring Harbor Laboratory, 1982. Alternatively, a gene library is transformed or transduced to E. coli, and then the gene library may be amplified to a large amount as described in Molecular Cloning, Cold Spring Harbor Laboratory, 1982.

A recombinant vector may be introduced into a host microorganism by a known method. For example, when using E. coli as a host microorganism, a calcium chloride method (Journal of Molecular Biology, Vol. 53, p. 159 (1970)), a rubidium chloride method (Methods in Enzymology, Vol. 68, p. 253 (1979)), electroporation (Current Protocols in Molecular Biology, Vol. 1, p. 184 (1994)) may be used. When using a cosmid vector or phage vector, transduction may be conducted using in vitro packaging (Current Protocols in Molecular Biology, Vol. 1, p. 571 (1994)). Alternatively, a method involving conjugational transfer may be used.

Then, a probe is prepared for obtaining a DNA fragment comprising a PHA synthase gene of P91 strain.

Some base sequences have been reported for PHA synthase genes; for example, Peoples, O. P. and Sinskey, A. J., J. Biol. Chem., 264, 15293 (1989); Huisman, G. W. et al., J. Biol. Chem., 266, 2191 (1991); Pieper, U. et al., FEMS Microbiol. Lett., 96, 73 (1992); Timm, A. and Steinbuchel, A., Eur. J. Biochem., 209, 15(1992); Matsusaki, H. et al., J. Bacteriol., 180, 6459 (1998).

From these reported sequences, a region where a sequence is preserved to a higher degree is selected for designing an oligonucleotide. Such an oligonucleotide includes, but not limited to, a sequence described in Timm, A. and Steinbuchel, A., Eur. J. Biochem., 209, 15 (1992). An oligonucleotide may be synthesized using, for example, Custom Synthesis Service, Amersham-Pharmacia Biotech.

Then, the designed oligonucleotide as a primer is subject to polymerase chain reaction (hereinafter, referred to as "PCR") using a chromosome DNA in P91 strain as a template to partially amplify the PHA synthase gene. The PCR amplified fragment thus obtained is homologous to the PHA synthase gene of P91 strain to about 100%, and may be expected to give a higher S/N ratio as a probe during colony hybridization and may allow stringency control in hybridization to be facilitated. The above PCR amplified fragment is labeled with an appropriate reagent and used for colony-hybridization of the above chromosome DNA library to select a PHA synthase gene (Current Protocols in Molecular Biology, Vol. 1, p. 603 (1994)). The PCR amplified fragment may be labeled using a commercially available kit such as AlkPhosDirect (Amersham-Pharmacia Biotech).

A gene fragment comprising a PHA synthase gene may be selected by, in addition to the above method using a genotype, a method using a phenotype where PHA synthesis is directly evaluated. The presence of PHA synthesis may be detected by, for example, staining with Sudan Black B (Archives of Biotechnology, VOl. 71, p. 283 (1970)) or determination of PHA accumulation by phase contrast microscopy.

A plasmid may be collected from E. coli selected by any of the above methods using an alkali method (Current Protocols in Molecular Biology, Vol. 1, p. 161 (1994)) to obtain a DNA fragment comprising a PHA synthase gene. The DNA fragment obtained may be sequenced by, for example, Sanger's sequencing method (Molecular Cloning, Vol. 2, p. 133 (1989). Alternatively, it may be conducted by a dye-primer method or a dye-terminator method using an automatic sequencer such as DNA Sequencer 377A (Parkin Elmer).

After determining all the sequences, hybridization may be conducted using a DNA fragment prepared by an appropriate method such as chemical synthesis, PCR using a chromosome DNA as a template or digestion a DNA fragment comprising the sequence with a restriction enzyme as a probe to provide a gene of this invention.

SEQ ID NOs.:2 and 4 show the sequences of PHA synthase gene of this invention while SEQ ID NOs.:1 and 3 show the amino acid sequences coded by the genes. As described above, there may be mutations for one or several amino acids such as deletion, substitution or addition as long as the polypeptides having these amino acid sequences retain PHA producing activity. In addition to those having the sequence coding the amino acids of SEQ ID NOs.:1 and 3, the present invention may include a degenerated isomer coding the same polypeptide which has the same amino acid sequence and is different only in a degeneration codon. Mutation such as deletion, substitution and addition may be introduced by, e.g., a site mutation introduction technique (Current Protocols in Molecular Biology Vol. 1, p. 811 (1994)).

A transformed microorganism of this invention may be produced by introducing a recombinant vector of the present invention into a host suitable to an expression vector used during preparing the recombinant vector. Examples of microorganisms which may be used as a host include various bacteria such as Esherichia sp., Pseudomonas sp., Ralstonia sp., Alcaligenes sp., Comamonas sp., Burkholderia sp., Agrobacterium sp., Flabobacterium sp., Vibrio sp., Enterobacter sp., Rhizobium sp., Gluconobacter sp., Acinetobacter sp., Moraxella sp., Nitrosomonas sp., Aeromonas sp., Paracoccus sp., Bacillus sp., Clostridium sp., Lactobacillus sp., Corynebacterium sp., Arthrobacter sp., Achromobacter sp., Micrococcus sp., Mycobacterium sp., Streptococcus sp., Streptomyces sp., Actinomyces sp., Norcadia sp. and Methylobacterium sp. Besides the above bacteria, yeasts and molds such as Saccharomyces sp. and Candida sp. may be used as a host.

When using a microorganism belonging to Pseudomonas sp., e.g., a bacterium such as E. coli as a host, it is preferable that the recombinant vector of the present invention itself can autonomously replicate in a host used while comprising a constitution required for expression such as a promoter, a DNA comprising a PHA synthase gene and a transcription termination sequence. Expression vectors include pLA2917 (ATCC 37355) having a RK2 replication origin which may be replicated and retained by a range of hosts or pJRD215 (ATCC 37533) having a RSF1010 replication origin, but any vector having a replication origin which may be replicated and retained by a wide range of hosts may be used.

Any promoter which may be expressed in a host may be used; for example, promoters derived from E. coli, a phage, etc. such as trp, trc, tac, lac, PL, PR, T7 and T3 promoters. A recombinant DNA may be introduced in a bacterium by an appropriate procedure such as the above calcium chloride method and electroporation.

When using a yeast as a host, an expression vector may be YEp13, YCp50, pRS or pYEX vector. A promoter may be, for example, GAL or AOD promoter. A recombinant DNA may be introduced into an yeast by, for example, electroporation (Methods Enzymol., 194, 182-187 (1990)), a spheroplast method (Proc. Natl. Acad. Sci. USA, 84, 1929-1933 (1978)) and a lithium acetate method (J. Bacteriol., 153, 163-168 (1983)).

A recombinant vector may further have a fragment for expressional regulation which has a variety of functions for suppression, amplification or triggering of expression; a marker for selection of a transformant; a resistance gene to an antibiotic; or a gene encoding a signal for extracellular secretion.

A PHA synthase of the present invention may be prepared by culturing a transformant prepared by transforming a host with a recombinant vector having a gene encoding the synthase to produce and accumulate a PHA synthase as a gene product in the culture (cultured bacterium or culture supernatant) and isolating the PHA synthase from the culture.

The transformant of the present invention may be cultured by a common process used for culturing a host.

Culturing may be conducted by any of common microorganism culturing processes such as batch, flow batch, continuous culturing and reactor styles.

For a transformant obtained using a bacterium such as E. coli as a host, a medium used for culturing may be a complete medium or synthetic medium such as LB medium and M9 medium. A microorganism may be grown by aerobically culturing it at a culturing temperature of 25 to 37°C for 8 to 72 hours to accumulate a PHA synthase in bacterial cells, and the enzyme may be collected. Microbial growth requires a carbon source including-sugars such as glucose, fructose, sucrose, maltose, galactose and starches; lower alcohols such as ethanol, propanol and butanol; polyalcohols such as glycerol; organic acids such as acetic acid, citric acid, succinic acid, tartaric acid, lactic acid and gluconic acid; and aliphatic acids such as propionic acid, butanoic acid, pentanoic acid, hexanoic acid, heptanoic acid, octanoic acid, nonanoic acid, decanoic acid, undecanoic acid and dodecanoic acid.

Examples of a nitrogen source include ammonia; ammonium salts such as ammonium chloride, ammonium sulfate and ammonium phosphate; and natural product derivatives such as peptone, meat extract, yeast extract, malt extract, casein decomposition products and corn steep liquor. Examples of an inorganic material include potassium dihydrogen phosphate, potassium monohydrogen phosphate, magnesium phosphate, magnesium sulfate and sodium chloride. The culture medium may contain an antibiotic such as kanamycin, ampicillin, tetracyclin, chloramphenicol and streptomycin.

When culturing a microorganism transformed using an expression vector having an inducible promoter, a proper inducer suitable to the type of the promoter may be added to a culture medium. For example, the inducer may be isopropyl-β-D-thiogalactopyranoside (IPTG), tetracyclin or indoleacrylic acid (IAA).

A PHA synthase may be separated and purified by centrifuging and collecting cells or a supernatant from a culture obtained and processing it by a technique such as cell disruption extraction, affinity chromatography, cation or anion exchange chromatography and gel filtration alone or in combination as appropriate. Whether a purified material is a desired enzyme may be determined by a usual method such as SDS polyacrylamide gel electrophoresis and Western blotting.

The present invention is not limited to the procedures as described above for culturing of a transformant using microorganism as a host, production of a PHA synthase by the transformant and accumulating it in microorganisms, and collection of the PHA synthase from the cells.

When culturing a transformant using a microorganism as a host for PHA production, the procedure may also be used in which the transformant is cultured using an appropriate medium composition and culturing conditions depending on factors such as the host used and the constitution of a recombinant vector introduced in the host and the PHA is obtained from the culture. A medium or culturing conditions may be the same as those illustrated for the above preparation of a PHA synthase.

A PHA may be collected from cells most conveniently by extraction with an organic solvent such as chloroform as usual, but in an environment where using an organic solvent such as chloroform is undesirable, the culture may be treated by a surfactant such as SDS, an enzyme such as lysozyme, or an agent such as EDTA, sodium hypochlorite and ammonia to remove bacterium components other than the PHA for collecting the PHA.

The present invention is not limited to the above procedures for culturing of a transformant using a microorganism as a host, production of a PHA by and accumulation thereof in the transformant, and collection of the PHA from the cells.

### Examples

The present invention will be more specifically described with reference to Examples although these Examples do not limit the technical range of this invention.

### Example 1: Cloning of a PHA synthase gene of P91 strain

P91 strain was cultured in 100 mL of LB medium (1% polypeptone, 0.5% yeast extract, 0.5% sodium chloride, pH 7.4) at 30°C overnight and then a chromosome DNA was separated and collected as described by Marmer. The chromosome DNA obtained was completely digested using a restriction enzyme BglII. A vector pUC18 was cleaned with a restriction enzyme BamHI. After dephosphorylation of the terminals (Molecular Cloning, Vol. 1, p. 572 (1989), Cold Spring Harbor Laboratory), the digested vector and the chromosome DNA fragment after BglII complete digestion were ligated using a DNA ligation kit Ver. II (Takara Shuzo Co., Ltd.). The ligated DNA fragment was used to transform Escheichia coli HB101 strain for preparing a chromosome DNA library for P91 strain.

Then, in order to select a DNA fragment comprising a PHA synthase gene of P91 strain, a probe was prepared. An oligonucleotide consisting of the sequences of SEQ ID NOs.:5 and 6 (Amersham-Pharmacia Biotech) was prepared and used as a primer for PCR. An amplified fragment was used as a probe. Labeling of the probe was conducted using AlkPhosDirect (Amersham-Pharmacia Biotech). The probe thus obtained was used to select an E. coli strain containing a recombinant plasmid comprising the PHA synthase gene from the chromosome DNA library of P91 strain by colony hybridization. From the selected strain, the plasmid was collected by an alkali method to prepare a DNA fragment comprising a PHA synthase gene.

The gene fragment thus obtained was recombined in a vector pBBR122 (Mo Bi Tec) comprising a wide host range of replication region which did not belong to IncP, IncQ or IncW in an incompatible group. The recombinant plasmid was transformed in Pseudomonas putida P91m1 strain (a strain depleted of PHA synthesizing ability) by electroporation, and then the P91m1 strain regained PHA synthesizing ability and exhibited complementarity.

The fragment comprising a PHA synthase gene was sequenced by Sanger's sequencing method. It was thus found that the fragment comprised a PHA synthase gene having the sequences of SEQ ID NOs.: 2 and 4. SEQ ID NOs.: 1 and 3 show the amino acid sequences coded by SEQ ID NOs.: 2 and 4, respectively.

### Example 2: Recombination of a PHA synthase gene of P91 strain to an expression vector

An oligonucleotide having a sequence around the initiation codon of the PHA synthase gene of SEQ ID NO.:2 (SEQ ID NO.:7) and an oligonucleotide having a sequence around the termination codon (SEQ ID NO.:8) were designed and synthesized (Amersham-Pharmacia Biotech). The oligonucleotides were used as a primer for PCR to amplify the whole length of the PHA synthase gene (LA-PCR kit; Takara Shuzo Co., Ltd.).

An oligonucleotide having a sequence around the initiation codon of the PHA synthase gene of SEQ ID NO.:4 (SEQ ID NO.:9) and an oligonucleotide having a sequence around the termination codon (SEQ ID NO.:10) were designed and synthesized (Amersham-Pharmacia Biotech). The oligonucleotides were used as a primer for PCR to amplify the whole length of the PHA synthase gene (LA-PCR kit; Takara Shuzo Co., Ltd.).

Each of the obtained PCR amplified fragment was completely digested using a restriction enzyme HindIII, and ligated to an expression vector pTrc99A which had been truncated with a restriction enzyme HindIII and dephosphorylated (Molecular Cloning, Vol. 1, p. 5.7.2 (1989), Cold Spring Harbor Laboratory), using a DNA ligation kit Ver. II (Takara Shuzo Co., Ltd.).

Using the recombinant plasmids, Escherichia coli HB101 was transformed by a calcium chloride method (Takara Shuzo Co., Ltd.), and recombinant plasmids collected from the transformants were designated as pP91-C1 (derived from SEQ ID NO.:2) and pP91-C2 (derived from SEQ ID NO.:4), respectively.

### Example 3: PHA production (1) using a PHA synthase gene recombinant E. coli

Using the recombinant plasmids obtained in Example 2, pP91-C1 (derived from SEQ ID NO.:2) and pP91-C2 (derived from SEQ ID NO.:4), an Escherichia coli HB101fB (fadB deficient strain) was transformed by a calcium chloride method to prepare recombinant E. coli strains derived from the recombinant plasmids, respectively.

Each of the pP91-C1 and pP91-C2 recombinant strains was inoculated to 200 mL of M9 medium containing 0.5% yeast extract and 0.1% FPVA, and the medium was shaken at 37°C with a rate of 125 strokes/min. After 24 hours, the cells were collected by centrifugation, washed once with cold methanol and lyophilized.

The lyophilized pellet was suspended in 100 mL of chloroform and the suspension was stirred at 60°C for 20 hours to extract a PHA. After filtering the extract through a membrane filter with a pore size of 0.45 pm, the filtrate was concentrated by rotary evaporation. Then, the concentrate was re-suspended in cold methanol and the precipitant was collected and dried in vacuo to provide a PHA. The PHA thus obtained was subject to methanolysis as usual and analyzed using a gas chromatography-mass spectrometry apparatus (GC-MS, Shimazu QP-5050, EI technique) to identify methyl-esterified PHA monomer units. The results are shown in Table 1.

**Table 1**

| | pP91-C1 recombinant strain | pP91-C2 recombinant strain |
|---|---|---|
| Cell dry weight | 810 mg/L | 800 mg/L |
| Polymer dry weight | 24 mg/L | 23 mg/L |
| Polymer dry weight/Cell dry weight | 3% | 3% |
| Monomer unit composition (area ratio) | | |
| 3-Hydroxybutyric acid | 0% | 0% |
| 3-Hydroxyvaleric acid | 0% | 0% |
| 3-Hydroxyhexanoic acid | 0% | 0% |
| 3-Hydroxyheptanoic acid | 5% | 3% |
| 3-Hydroxyoctanoic acid | 4% | 5% |
| 3-Hydroxynonanoic acid | 9% | 11% |
| 3-Hydroxydecanoic acid | 10% | 12% |
| 3-Hydroxy-5-(4-fluorophenyl) valeric acid | 72% | 69% |

### (Example 4: PHA production (2) using a PHA synthase gene recombinant E. coli)

Each of the pP91-C1 and pP91-C2 recombinant strains was inoculated to 200 mL of M9 medium containing 0.5% yeast extract and 0.2% PxBA, and then cultured with shaking at 37°C with a rate of 125 strokes/min. After 24 hours, the cells were collected by centrifugation, washed once with cold methanol and lyophilized.

The lyophilized pellet was suspended in 100 mL of chloroform and the suspension was stirred at 60°C for 20 hours to extract a PHA. After filtering the extract through a membrane filter with a pore size of 0.45 µm, the filtrate was concentrated by rotary evaporation. Then, the concentrate was re-suspended in cold methanol and only the precipitant was collected and dried in vacuo to provide a PHA. The PHA thus obtained was subject to methanolysis as usual and analyzed using a gas chromatography-mass spectrometry apparatus (GC-MS, Shimazu QP-5050, EI technique) to identify methyl-esterified PHA monomer units. The results are shown in Table 2.

**Table 2**

| | pP91-C1 recombinant strain | pP91-C2 recombinant strain |
|---|---|---|
| Cell dry weight | 750 mg/L | 720 mg/L |
| polymer dry weight | 4 mg/L | 4 mg/L |
| Polymer dry weight/Cell dry weight | 0.5% | 0.6% |
| Monomer unit composition (area ratio) | | |
| 3-Hydroxybutyric acid | 0% | 0% |
| 3-Hydroxyvaleric acid | 0% | 0% |
| 3-Hydroxyhexanoic acid | 0% | 0% |
| 3-Hydroxyheptanoic acid | 2% | 2% |
| 3-Hydroxyoctanoic acid | 3% | 3% |
| 3-Hydroxynonanoic acid | 5% | 7% |
| 3-Hydroxydecanoic acid | 5% | 6% |
| 3-Hydroxy-4-phenoxy-n-butyric acid | 85% | 82% |

## Claims

1. A polyhydroxyalkanoate synthase having the amino acid sequence of SEQ ID NO. 1.

2. A polyhydroxyalkanoate synthase having an amino acid sequence wherein the amino acid sequence of SEQ ID NO. 1 has been modified by deleting, substituting or adding amino acids to such extent that the resulting polyhydroxyalkanoate synthase still has the activity to convert 5(4-fluorophenyl)valeric acid into a polymer comprising 3-hydroxy-5-(4-fluorophenyl)valeric acid as a monomer unit.

3. A polyhydroxyalkanoate synthase having an amino acid sequence wherein the amino acid sequence of SEQ ID NO. 1 has been modified by deleting, substituting or adding amino acids to such extent that the resulting polyhydroxyalkanoate synthase still has the activity to convert 4-phenoxy-n-butyric acid into a polymer comprising 3-hydroxy-4-phenoxy-n-butyric acid as a monomer unit.

4. A polyhydroxyalkanoate synthase gene comprising a DNA sequence encoding the amino acid sequence of the polyhydroxyalkanoate synthase according to claim 2 or 3.

5. The polyhydroxyalkanoate synthase gene comprising a DNA sequence of SEQ ID NO. 2 encoding the amino acid sequence of SEQ ID NO. 1.

6. A polyhydroxyalkanoate synthase having the amino acid sequence of SEQ ID NO. 3.

7. A polyhydroxyalkanoate synthase having an amino acid sequence wherein the amino acid sequence of SEQ ID NO. 3 has been modified by deleting, substituting or adding amino acids to such extent that the resulting polyhydroxyalkanoate synthase still has the activity to convert 5(4-fluorophenyl)valeric acid into a polymer comprising 3-hydroxy-5-(4-fluorophenyl)valeric acid as a monomer unit.

8. A polyhydroxyalkanoate synthase having an amino acid sequence wherein the amino acid sequence of (SEQ ID NO. 3 has been modified by deleting, substituting or adding amino acids to such extent that the resulting polyhydroxyalkanoate synthase still has the activity to convert 4-phenoxy-n-butyric acid into a polymer comprising 3-hydroxy-4-phenoxy-n-butyric acid as a monomer unit.

9. A polyhydroxyalkanoate synthase gene comprising a DNA sequence encoding the amino acid sequence of the polyhydroxyalkanoate synthase according to claim 7 or 8.

10. The polyhydroxyalkanoate synthase gene comprising a DNA sequence of SEQ ID NO. 4 encoding the amino acid sequence of SEQ ID NO. 3.

11. A recombinant vector comprising the gene according to claim 4, 5, 9 or 10 as a polyhydroxyalkanoate synthase gene.

12. A transformed microorganism transformed by introduction of the recombinant vector according to claim 11.

13. A method for preparing a polyhydroxyalkanoate comprising the steps of culturing the transformed microorganism according to claim 12 in a medium containing a substrate for a polyhydroxyalkanoate synthase and isolating the polyhydroxyalkanoate from the culture obtained.

14. A method for producing a polyhydroxyalkanoate synthase comprising the steps of culturing the transformed microorganism according to claim 12 and making the transformed microorganism produce the polyhydroxyalkanoate synthase.

## Patentansprüche

1. Polyhydroxyalkanoat-Synthase, die die Aminosäuresequenz der Sequenzidentifizierungsnr. 1 aufweist.

2. Polyhydroxyalkanoat-Synthase mit einer Aminosäuresequenz, wobei
die Aminosäuresequenz der Sequenzidentifizierungsnr. 1 modifiziert worden ist, indem Aminosäuren so weit deletiert, substituiert oder hinzugefügt wurden, dass die entstehende Polyhdydroxyalkanoat-Synthase noch immer die Aktivität hat, 5(4-Fluorphenyl)valeriansäure in ein Polymer zu überführen, das 3-Hydroxy-5-(4-fluorphenyl)valeriansäure als eine Monomereinheit aufweist.

3. Polyhydroxyalkanoat-Synthase mit einer Aminosäuresequenz, wobei
die Aminosäuresequenz der Sequenzidentifizierungsnr. 1 modifiziert worden ist, indem Aminosäuren so weit deletiert, substituiert oder hinzugefügt wurden, dass die entstehende Polyhdydroxyalkanoat-Synthase noch immer die Aktivität hat, 4-Phenoxy-n-buttersäure in ein Polymer zu überführen, das 3-Hydroxy-4-phenoxy-n-buttersäure als eine Monomereinheit aufweist.

4. Polyhydroxyalkanoat-Synthase-Gen, umfassend:
eine DNA-Sequenz, die die Aminosäuresequenz der Polyhydroxyalkanoat-Synthase nach Anspruch 2 oder 3 codiert.

5. Polyhydroxyalkanoat-Synthase-Gen, umfassend:
eine DNA-Sequenz der Sequenzidentifizierungsnr. 2, die die Aminosäuresequenz der Sequenzidentifizierungsnr. 1 codiert.

6. Polyhydroxyalkanoat-Synthase mit der Aminosäuresequenz der Sequenzidentifizierungsnr. 3.

7. Polyhydroxyalkanoat-Synthase mit einer Aminosäuresequenz, wobei
die Aminosäuresequenz der Sequenzidentifizierungsnr. 3 modifiziert worden ist, indem Aminosäuren so weit deletiert, substituiert oder hinzugefügt wurden, dass die entstehende Polyhdydroxyalkanoat-Synthase noch immer die Aktivität hat, 5(4-Fluorphenyl)valeriansäure in ein Polymer zu überführen, das 3-Hydroxy-5-(4-fluorphenyl)valeriansäure als eine Monomereinheit aufweist.

8. Polyhydroxyalkanoat-Synthase mit einer Aminosäuresequenz, wobei
die Aminosäuresequenz der Sequenzidentifizierungsnr. 3 modifiziert worden ist, indem Aminosäuren so weit deletiert, substituiert oder hinzugefügt wurden, dass die entstehende Polyhdydroxyalkanoat-Synthase noch immer die Aktivität hat, 4-Phenoxy-n-buttersäure in ein Polymer zu überführen, das 3-Hydroxy-4-phenoxy-n-buttersäure als eine Monomereinheit aufweist.

9. Polyhydroxyalkanoat-Synthase-Gen, umfassend:
eine DNA-Sequenz, die die Aminosäuresequenz der Polyhydroxyalkanoat-Synthase nach Anspruch 7 oder 8 codiert.

10. Polyhydroxyalkanoat-Synthase-Gen, umfassend:
eine DNA-Sequenz der Sequenzidentifizierungsnr. 4, die die Aminosäuresequenz der Sequenzidentifizierungsnr. 3 codiert.

11. Rekombinant-Vektor, umfassend:
das Gen nach Anspruch 4, 5, 9 oder 10 als ein Polyhydroxyalkanoat-Synthase-Gen.

12. Transformierter Mikroorganismus, der durch Einführen des Rekombinant-Vektors nach Anspruch 1 transformiert ist.

13. Verfahren zum Herstellen eines Polyhydroxyalkanoats, umfassend die Schritte:
- Züchten des transformierten Mikroorganismus nach Anspruch 12 in einem Medium, das ein Substrat für eine Polyhydroxyalkanoat-Synthase enthält, und
- Abtrennen des Polyhydroxyalkanoats von der erhaltenen Kultur.

14. Verfahren zum Herstellen einer Polyhydroxyalkanoat-Synthase, umfassend die Schritte:
- Züchten des transformierten Mikroorganismus nach Anspruch 1 2 und
- Bewirken, dass der transformierte Mikroorganismus die Polyhydroxyalkanoat-Synthase produziert.

## Revendications

1. Polyhydroxyalcanoate-synthase ayant la séquence d'aminoacides de la SEQ ID N° 1.

2. Polyhydroxyalcanoate-synthase ayant une séquence d'aminoacides dans laquelle la séquence d'aminoacides de la SEQ ID N° 1 a été modifiée par délétion, substitution ou addition d'aminoacides de telle sorte que la polyhydroxyalcanoate-synthase résultante ait encore l'activité de conversion de l'acide 5(4-fluorophényl)valérique en un polymère comprenant de l'acide 3-hydroxy-5-(4-fluorophényl)-valérique comme motif monomère.

3. Polyhydroxyalcanoate-synthase ayant une séquence d'aminoacides dans laquelle la séquence d'aminoacides de la SEQ ID N° 1 a été modifiée par délétion, substitution ou addition d'aminoacides de telle sorte que la polyhydroxyalcanoate-synthase résultante ait encore l'activité de conversion de l'acide 4-phénoxy-n-butyrique en un polymère comprenant de l'acide 3-hydroxy-4-phénoxy-n-butyrique comme motif monomère.

4. Gène de polyhydroxyalcanoate-synthase comprenant une séquence d'ADN codant pour la séquence d'aminoacides de la polyhydroxyalcanoate-synthase suivant la revendication 2 ou 3.

5. Gène de polyhydroxyalcanoate-synthase comprenant une séquence d'ADN de la SEQ ID N° 2 codant pour la séquence d'aminoacides de la SEQ ID N° 1.

6. Polyhydroxyalcanoate-synthase ayant la séquence d'aminoacides de la SEQ ID N° 3.

7. Polyhydroxyalcanoate-synthase ayant une séquence d'aminoacides dans laquelle la séquence d'aminoacides de la SEQ ID N° 3 a été modifiée par délétion, substitution ou addition d'aminoacides de telle sorte que la polyhydroxyalcanoate-synthase résultante ait encore l'activité de conversion de l'acide 5(4-fluorophényl)valérique en un polymère comprenant de l'acide 3-hydroxy-5-(4-fluorophényl)-valérique comme motif monomère.

8. Polyhydroxyalcanoate-synthase ayant une séquence d'aminoacides dans laquelle la séquence d'aminoacides de la SEQ ID N° 3 a été modifiée par délétion, substitution ou addition d'aminoacides de telle sorte que la polyhydroxyalcanoate-synthase résultante ait encore l'activité de conversion de l'acide 4-phénoxy-n-butyrique en un polymère comprenant de l'acide 3-hydroxy-4-phénoxy-n-butyrique comme motif monomère.

9. Gène de polyhydroxyalcanoate-synthase comprenant une séquence d'ADN codant pour la séquence d'aminoacides de la polyhydroxyalcanoate-synthase suivant la revendication 7 ou 8.

10. Gène de polyhydroxyalcanoate-synthase comprenant une séquence d'ADN de la SEQ ID N° 4 codant pour la séquence d'aminoacides de la SEQ ID N° 3.

11. Vecteur recombinant comprenant le gène suivant la revendication 4, 5, 9 ou 10 comme gène de polyhydroxyalcanoate-synthase.

12. Microorganisme transformé, qui a été transformé par introduction du vecteur recombinant suivant la revendication 11.

13. Procédé pour la préparation d'un polyhydroxyalcanoate, comprenant les étapes consistant à cultiver le microorganisme transformé suivant la revendication 12 dans un milieu contenant un substrat pour une polyhydroxyalcanoate-synthase, et à isoler le polyhydroxyalcanoate de la culture obtenue.

14. Procédé pour la production d'une polyhydroxyalcanoate-synthase, comprenant les étapes consistant à cultiver le microorganisme transformé suivant la revendication 12 et à amener le microorganisme transformé à produire la polyhydroxyalcanoate-synthase.
